# FASCICULE DE BREVET EUROPEEN

(11) **EP 2 616 119 B1**
(45) Date de publication et mention de la délivrance du brevet: **03.05.2017**
(21) Numéro de dépôt: 11760458.7
(22) Date de dépôt: 16.09.2011
(51) Int. Cl.: A61M 5/00, B01L 9/00, B01L 9/06, B65D 21/02, B65D 43/02, B65D 71/00, B65D 77/00, B65D 81/00

(54) **CONDITIONNEMENT POUR LE STOCKAGE, LA PROTECTION ET LE TRANSPORT DE SERINGUES**
AUFBEWAHRUNGSSYSTEM ZUM LAGERN, ZUM SCHUTZ UND ZUM TRANSPORT VON SPRITZEN
SUPPORT UNIT FOR STORAGE, PROTECTION AND TRANSPORT OF SYRINGES

(30) Priorité: 30.03.2011 BE 201100195; 17.09.2010 BE 201000554
(43) Date de publication de la demande: 24.07.2013
(73) Titulaire: Flexiways Sprl, 6723 Habay-la-Vieille (BE)
(72) Inventeur: LEPOT, Eric, B-1348 Louvain-la-Neuve (BE)
(74) Mandataire: Pecher, Nicolas
(86) Numéro de dépôt international: PCT/EP2011/066145
(87) Numéro de publication internationale: WO 2012/035155

(56) Documents cités:
- EP-A1- 1 088 566
- EP-A1- 2 072 071
- WO-A1-95/08392
- WO-A2-94/14484
- US-A- 1 916 859
- US-A- 2 818 979

## Description

### DOMAINE TECHNIQUE

La présente invention concerne un conditionnement pour le stockage, la protection et le transport de contenants, plus particulièrement de contenants suspendus en position verticale tels que par exemple des seringues.

### ARRIERE-PLAN TECHNOLOGIQUE

Actuellement, les contenants de produits médicaux, tels que des seringues en verre, sont conditionnés par les fabricants dans une forme appelée « nest » et livrés dans des conditions stériles. Livrer des seringues sous un format « nest » signifie livrer des seringues qui ont été emballées sur un plateau en plastique comprenant principalement une plaque de support, nommé ci-après plateau-nest, qui est formé d'une matrice d'ouvertures, chacune de ces ouvertures supportant une seringue. Ce plateau-nest est typiquement supporté par une boîte en plastique nommé, ci-après, boîte nest qui protège les seringues (voir par exemple le produit **HYPAK^{®} SCF^{®},** Becton, Dickinson and Company of Franklin Lakes, New Jersey, USA). Ce conditionnement est, de plus, recouvert par un opercule, mis en sachet, et est alors prêt pour la stérilisation.

Quand les seringues sont livrées sous le format « nest » à une société pharmaceutique, elles doivent être manipulées pour être remplies une à une. Après avoir enlevé le sachet et l'opercule de chaque conditionnement, manuellement ou au moyen d'équipements automatisés, chaque plateau-nest est retiré de sa boîte nest correspondante et les seringues sont alors remplies une à une, ligne par ligne ou par série de plusieurs pièces. Après l'étape de remplissage, il est nécessaire d'une part, d'étiqueter chaque seringue dans le but de définir le produit médical contenu et d'autre part, d'inspecter les seringues pour détecter toute contamination du produit ainsi que les fissures ou griffes éventuelles dans les seringues. D'autres opérations peuvent être requises comme par exemple l'ajout de protections d'aiguilles ou de tiges de piston. L'inspection est classiquement réalisée au moyen d'un système de capteurs optiques (ou d'une caméra) qui inspectent une par une les seringues. Cependant, en accord avec la pratique habituelle et en raison de limitations techniques (quantité de seringues, difficultés à étiqueter et/ou à inspecter les seringues placées dans la partie centrale du plateau-nest...etc.), les seringues sont d'abord enlevées du plateau-nest avant d'être étiquetées et/ou contrôlées. Ainsi, lorsque les seringues sont finalement étiquetées et /ou contrôlées, elles sont alors encore manipulées et stockées soit directement dans la boîte nest sans être stockées sur le plateau-nest, soit dans un support différent, nommé, ci-après plateau-peigne (voir le document US 6,012,595 décrit ci-après), qui possède de multiples doigts allongés pour garder les seringues suspendues par leurs collerettes. Il doit être remarqué qu'après l'étiquetage et/ou l'inspection, les seringues ne sont plus réinsérées dans le plateau-nest parce qu'il est plus efficient et facile de les stocker dans le plateau-peigne qui est acheté séparément. Un conditionnement de type nest, tel que le produit **HYPAK^{®} SCF^{®}**, n'est donc pas adapté pour une manipulation automatisée car il requiert des machines complexes. D'autre part, dans le cadre d'une manipulation semi-automatique (avec assistance humaine), il est difficile de dépasser les cadences de plus de 6000 seringues par heure. Cependant, si d'une part, les plateaux-peignes sont préférables aux plateaux-nests pour stocker les seringues de façon plus rapide et plus aisée, ils ne sont, d'autre part, pas adaptés au transport et au stockage des seringues. En effet, quand les plateaux-peignes sont stockés les uns sur les autres en colonnes verticales, certaines parties des seringues risquent d'être endommagées ou brisées, en raison de leur contact direct avec le dessous du conditionnement posé au-dessus. Afin d'éviter ce problème, les plateaux-peignes doivent être placés dans des boîtes spécifiques supplémentaires et munis de couvercles avec un rebord intérieur de blocage.

Des boîtes spécifiques dans lesquelles sont disposés les contenants médicaux sont connues dans l'art mais celles-ci ne sont pas adaptées, du fait de leur structure, à toutes les étapes automatisées de la chaîne de production. Ainsi, une boîte ayant une structure trop souple, pas assez rigide, empêchera par exemple la bonne efficacité d'une étape de palettisation ou de dé-palettisation puisque celle-ci seront difficilement manipulable par un système automatisé.

Plusieurs modes de stérilisation sont communément utilisés pour traiter les contenants avant leur remplissage: la stérilisation par vapeur, la stérilisation par un gaz tel que l'ETO et la stérilisation au moyen de rayonnement ionisant. Pour la stérilisation par vapeur, il est nécessaire de retourner les contenants, de telle sorte que l'eau de condensation qui se déposerait à l'intérieur du contenant puisse s'écouler par gravité. Lors de la stérilisation par gaz ETO, il est également nécessaire de pouvoir retourner les contenants afin de favoriser l'évacuation de résidus de gaz ETO, plus lourd que l'air. Il est donc nécessaire de prévoir des moyens qui maintiennent les contenants dans leur plateau-nest en position retournée.

Les règles en matière de sécurité des produits médicaux imposent que la présence de particules ou de poussières soit réduite au minimum. Dans les systèmes connus, des frottements entres divers composants de la boîte lors du transport ou du stockage peuvent provoquer la création de telles particules. Elles imposent également d'éviter que lors de chocs, les contenant soient griffés ou fragilisés. On cherche également à éviter la manipulation humaine et même la présence d'opérateurs humains à proximité des contenants en fabrication. En somme, les conditionnements de stockage et de transport visent à protéger la seringue de tout dommage ou contamination lors des opérations industrielles de production, transport et/ou stockage.

### ETAT DE LA TECHNIQUE

Le dispositif de transport, de stockage et de protection de seringues proposé par la demanderesse et décrit dans le document WO 2009/053434 vise à répondre aux besoins précités. Il propose en particulier un plateau 30 présentant une pluralité de rails 31 formant des gorges 32 et permettant l'insertion/déchargement aisé de contenants, et une paroi supérieure 34 qui maintient les contenants lors du retournement du plateau. Cependant, l'usage de plateaux-nests constitue un standard industriel largement implanté. Il existe donc un besoin pour une solution aux problèmes précités qui soit compatible avec ce standard industriel ;

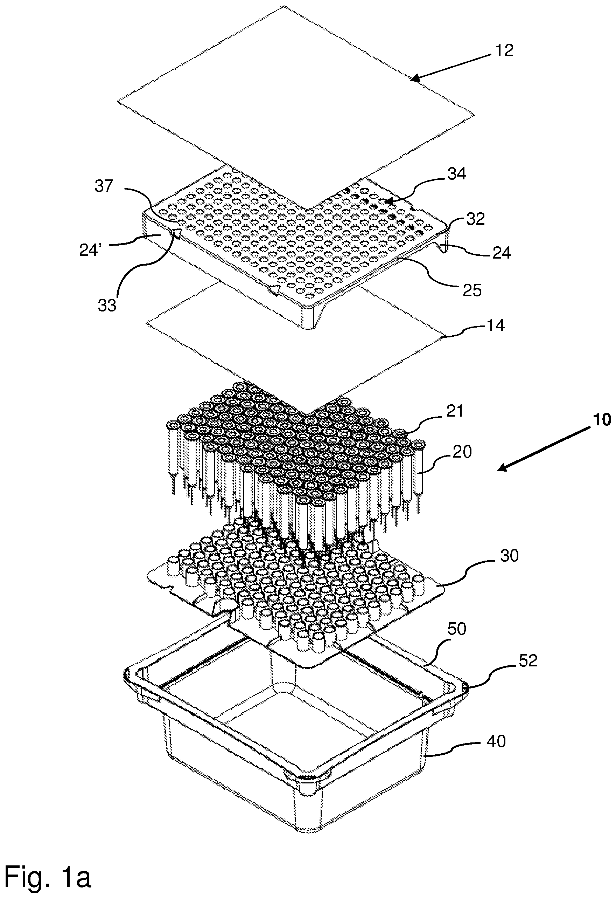

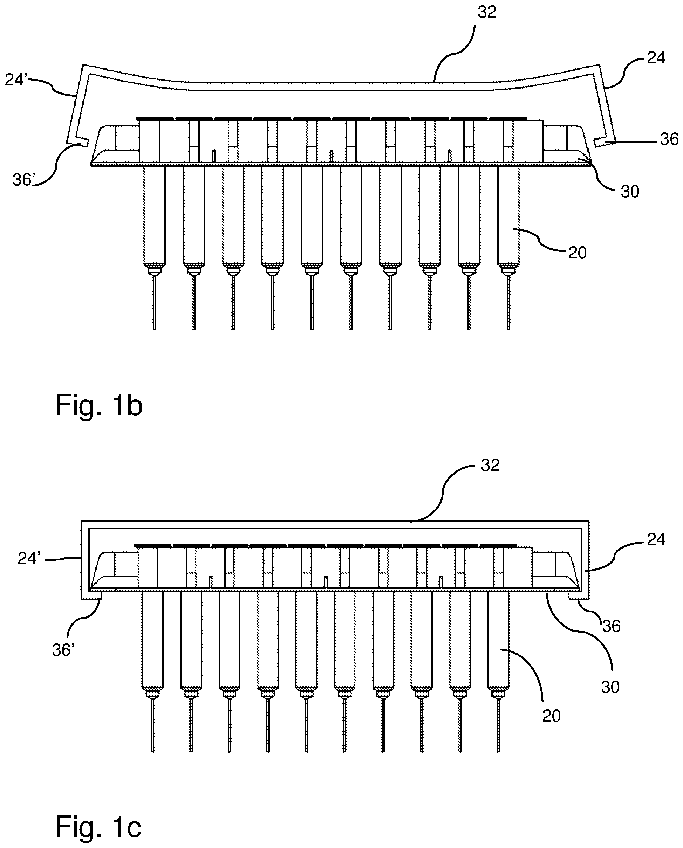

Il est connu par le document EP 1088566 un dispositif de manutention d'objets. En référence à la Fig. 10 de ce document, une boîte 1 comporte un couvercle 4, et des moyens pour maintenir et guider des seringues dans la boîte. Le couvercle 4 est articulé au moyen d'une charnière et permet d'éviter que les seringues ne sortent de la boîte lors de la manipulation, en particulier lors du retournement pour la stérilisation à la vapeur. Ce dispositif est utile pour l'étape de stérilisation, mais ne convient pas pour contenir des seringues tout au long de la chaine de fabrication, y compris le transport et le stockage. Il nécessite de sortir les contenants du plateau-nest original pour les mettre dans les rainures traversantes (élément 26 de la Fig. 1). Les contenants sont alors susceptibles de se toucher, créant ainsi des particules et risquant également de fragiliser les contenants.

On connait par le document EP 2072071 un couvercle de plateau-nest. Ce couvercle 10 est posé sur la plaque basale 2 du plateau-nest 1. Des leviers de commande 17 permettent d'orienter des ailes 16 en position de préhension, ce qui a pour effet de solidariser le couvercle 10 au plateau-nest 1, et de permettre alors la manipulation du plateau-nest. Cependant, ce dispositif n'est pas conçu pour le traitement automatique : lorsque le plateau-nest 1 est placé dans une boîte 8, rien n'est prévu pour permettre la préhension du plateau-nest par une pince de robot. De plus, les leviers de commande interdisent la superposition de plusieurs boîtes munies d'un tel couvercle. En effet, le levier dépasse le plan formé par le rebord du capot. Un tel couvercle comporte de nombreuses pièces mobiles et est de préférence réalisé en acier inoxydable. Son coût de fabrication, bien qu'il autorise son utilisation pour l'étape de stérilisation, ne permet pas de l'utiliser à plus grande échelle lors du transport et du stockage. Enfin, les nest utilisés dans l'industrie se présentent sous différents formats: les nests pour 160 contenants présentent les « orifices pour passages de doigts 7 » (également connus sons le nom d' « oreilles ») sur le côté long, alors que les nests conçus pour 100 contenants présentent ces orifices sur le côté court. Ceci implique que les moyens de manipulation tels que les racks utilisés dans l'autoclave soient différents pour chaque type de nest, c.à.d. pour chaque format, dans toute la chaîne de production.

On connaît également le document US 2,818,979 décrivant un dispositif pour maintenir des seringues. Le dispositif comprend un plateau et un couvercle, sans flancs latéraux, pouvant être maintenus ensemble par l'intermédiaire d'un clip. Le document WO 94/14484 décrit un conditionnement comprend un plateau de type peigne et un couvercle. Les bords du plateau sont disposés sur un rebord de la boîte empêchant les flancs du couvercle de venir s'agencer sous le plateau.

### RÉSUMÉ DE L'INVENTION

L'invention a pour but de remédier aux inconvénients précités. En particulier, elle vise à fournir un conditionnement qui permette une immobilisation des contenants dans un plateau-nest, qui évite ainsi les risques de création de particules, et qui permette la stérilisation à la vapeur en position retournée, tout en autorisant une manipulation aisée par des moyens automatiques tels que des robots et qui soit compatible avec les standards industriels, quel que soit le format du plateau-nest. L'invention vise également à fournir des conditionnements superposables.

Suivant un premier aspect, l'invention se rapporte à un conditionnement pour le stockage et/ou le transport et/ou le traitement et/ou le support dans une position verticale suspendue d'objets qui présentent des flasques. Ce conditionnement comprend un plateau, apte à maintenir une pluralités des dits objets dans une position verticale, et un couvercle apte à recouvrir le dit plateau. Suivant l'invention ce couvercle comprend une paroi supérieure et au moins deux flancs s'étendant vers le bas de deux cotés opposés de la paroi supérieure, chacun des flancs étant prolongés, à leurs extrémités inférieures, de doigts aptes à s'agencer sous une face inférieure dudit plateau, solidarisant ainsi le plateau et le couvercle.

La paroi supérieure présente avantageusement une flexibilité permettant de l'incurver de manière à écarter un doigt d'un doigt opposé, et permettre ainsi l'agencement de ces doigts sous la face inférieure dudit plateau. Le couvercle peut ainsi être présenté par le dessus, sous forme infléchie, puis relâché pour se solidariser au plateau.

La paroi supérieure du couvercle peut présenter une pluralité de passages. Ces passages peuvent permettre la circulation de gaz et/ou le remplissage des contenants.

La paroi supérieure peut également comporter sur sa face inférieure, des nervures. Ces nervures, qui assurent la rigidité sont de préférence parallèles aux flancs, et de préférence au dessus des collerettes.

Dans un second mode préféré de l'invention, la paroi supérieure comporte sur sa face inférieure, au moins deux parois de support s'étendant vers le bas, parallèlement aux flancs et situés du coté intérieur de ceux-ci. Ces parois de support permettent de poser le couvercle sur le plateau, de préférence à hauteur du U inversé du redan, et facilitent ainsi l'empilement de plusieurs conditionnements.

Dans un troisième mode préféré de l'invention, les flancs sont interrompus, et des portions de tube sont agencées dans ces interruptions. Ces portions de tube permettent de poser le couvercle sur le plateau.

Le conditionnement suivant l'invention peut en outre comprendre une boîte avec une ouverture supérieure, un fond, des parois latérales et un redan formé entre ladite ouverture supérieure et lesdits parois latérales pour supporter ledit plateau. Suivant l'invention, ce redan comporte une nervure en forme de « U » inversé, s'étendant sur la portion intérieure dudit redan. Cette nervure offre un dégagement qui permet la pose du couvercle sur un plateau disposé dans une boîte.

Des premiers moyens d'immobilisation d'un plateau dans une boîte prévoient que le redan comporte au moins un plot de centrage et que le plateau comporte au moins une échancrure ou un trou rond correspondant.

Les plots ont avantageusement une forme tronconique. Le ou les échancrures peuvent présenter une forme en « V ».

Suivant d'autres moyens d'immobilisation, la face interne de la paroi latérale supérieure de la boîte comporte une nervure verticale. Cette nervure guide et bloque le plateau dans la boîte.

La boîte comporte un rebord supérieur qui peut avantageusement comporter, dans au moins un angle de la boîte une ouverture. Cette ouverture permet l'élimination de la condensation et de l'ETO et permet également de soulever un éventuel opercule collé.

Suivant un second aspect, l'invention se rapporte à un procédé de stockage et/ou de transport et/ou de traitement et/ou de support dans une position verticale suspendue d'objets qui présentent des flasques, comprenant l'utilisation d'un plateau, apte à maintenir une pluralités des dits objets dans une position verticale, et un couvercle apte à recouvrir le dit plateau, comportant les étapes de
- mise à disposition d'un couvercle comprenant une paroi supérieure et au moins deux flancs s'étendant vers le bas de deux cotés opposés de la paroi supérieure, chacun des flancs étant prolongé, à son extrémité inférieure, de doigts aptes à s'agencer sous une face inférieure dudit plateau,
- préhension du dit couvercle par des moyens de préhension ;
- imposition par les moyens de préhension d'une courbure à la paroi supérieure du couvercle écartant deux flancs l'un de l'autre ;
- positionnement du couvercle en regard du plateau;
- relâchement de la dite courbure, les doigts solidarisant ainsi le plateau et le couvercle.

De préférence, l'étape de positionnement du couvercle en regard du plateau est réalisée, le plateau étant supporté dans une boîte.

Suivant un troisième aspect, une méthode de séparation et/ou d'assemblage des éléments constitutifs d'un ou plusieurs conditionnements selon la présente invention est fournie. Chaque conditionnement comprend un plateau, apte à maintenir une pluralité d'objets munis de flasques dans une position verticale, un couvercle apte à recouvrir ledit plateau, et une boîte supportant ledit plateau. Ladite méthode de séparation et/ou d'assemblage comporte les étapes de :
- préhension d'un ou plusieurs conditionnements selon l'invention par un moyen de préhension automatique,
- séparation de ladite boîte de l'ensemble formé par le plateau recouvert du couvercle par l'intermédiaire dudit moyen de préhension automatique,
- séparation dudit plateau contenant lesdits objets dudit couvercle par l'intermédiaire dudit moyen de préhension automatique,
- traitement dudit plateau,
- réassemblage successif dudit plateau avec ledit couvercle puis de l'ensemble ainsi formé avec ladite boîte par l'intermédiaire dudit moyen de préhension automatique.

De préférence, l'étape de traitement dudit plateau peut être le retrait des objets maintenus en position verticale dans ledit plateau pour mettre ceux-ci à disposition. Alternativement, l'étape de traitement dudit plateau peut être la disposition d'objets munis de flasques dans ledit plateau. Ledit plateau peut être un plateau de type nest ou en forme de peigne.

De préférence, le moyen de préhension automatique peut être un robot de préhension. Ledit robot de préhension peut être muni d'un seul préhenseur. Ledit préhenseur est apte à séparer et assembler la boîte, le plateau et le couvercle des conditionnements selon la présente invention.

De préférence, ladite méthode peut utiliser un couvercle comprenant une paroi supérieure et au moins deux flancs s'étendant vers le bas de deux cotés opposés de la paroi supérieure, chacun des flancs étant prolongés, à leurs extrémités inférieures, de doigts aptes à s'agencer sous une face inférieure dudit plateau, solidarisant ainsi le plateau et le couvercle, et en ce que lesdits flancs sont interrompus, et que des portions de tube sont agencés dans ces interruptions.

De préférence, ladite méthode de séparation peut être effectuée avec au moins deux conditionnements simultanément. En particulier, ladite méthode de séparation peut être effectuée avec deux conditionnements simultanément. Ladite méthode selon l'invention permet ainsi la séparation ou l'assemblage des éléments constitutifs d'un conditionnement selon l'invention dans une chaîne de production à l'aide d'un moyen de préhension automatique. La méthode est facilitée grâce aux caractéristiques techniques du couvercle tel que décrit ci-dessus. En particulier, ladite méthode peut être favorisée lorsque le couvercle est muni de flancs sur deux côtés de celui-ci, lesdits flancs sont interrompus et agencés à des portions de tube. En effet, une telle méthode peut permettre la préhension automatique du conditionnement dans des conditions optimales sans risque de détérioration desdits objets contenus dans le plateau, même en position verticale. Grâce aux caractéristiques du couvercle selon l'invention, un moyen de préhension automatique muni d'un préhenseur unique peut assembler ou dissocier l'ensemble du conditionnement. Ladite méthode présente donc de nombreux avantages susceptibles d'applications dans le domaine de la logistique interne d'une entreprise (ou transitique), c'est à dire l'ensemble des opérations permettant le convoyage, le transfert et la manutention de matières, et de produits.

### BRÈVE DESCRIPTION DES DESSINS

La Fig. 1 a est une vue en perspective éclatée de composants d'un conditionnement suivant un premier mode de réalisation de l'invention.
Les Figs. 1b et 1c sont des schémas de principe d'un plateau et d'un couvercle d'un conditionnement suivant l'invention. Dans la Fig. 1b, le couvercle est recourbé en vue de son insertion sur le plateau. Dans la Fig. 1c, le couvercle est agencé sur le plateau.
Les Figs. 2a et 2b sont respectivement, une vue en coupe suivant le plan AA indiqué sur la Fig. 2b et une vue de dessus de ce même conditionnement.
Les Figs. 2c et 2d sont des vues agrandies des détails indiqués par les cercles « C » et « D » sur la Fig. 2a.
La Fig. 2e est semblable la Fig. 2d, dans un second mode de réalisation de l'invention.
La Fig. 3a est une vue en perspective cavalière et en coupe d'une partie d'un conditionnement suivant un troisième mode de réalisation de l'invention.
La Fig. 3b est une vue de dessous d'un couvercle d'un conditionnement suivant ce troisième mode de réalisation.
La Fig. 4a est une vue du dessus d'un boite contenant un plateau d'un conditionnement suivant l'invention, et représentant des premiers moyens d'immobilisation du plateau.
Les Fig. 4b, 4c, 4d sont des vues en perspective, agrandie, d'une portion de la vue de la Fig. 4a représentant respectivement des premiers, second et troisièmes moyens d'immobilisation du plateau.
La Fig. 5 représente une vue schématique d'une zone de manipulation d'un conditionnement selon un mode de réalisation suivant l'invention.
La Fig. 6 représente une vue en perspective d'un couvercle comprenant des moyens de positionnement d'un second conditionnement selon un mode de réalisation de l'invention.

### DESCRIPTION DÉTAILLÉE DE L'INVENTION

L'invention est décrite ci-après par des exemples, en relation avec les figures, dans lesquelles des éléments identiques ou correspondants sont référencés par les mêmes numéros.

La Fig. 1 a est une vue en perspective éclatée de composants d'un conditionnement 10 suivant un premier mode de réalisation de l'invention et représente successivement, de haut en bas, un opercule 12 qui peut être utilisé pour fermer le conditionnement, un couvercle 32, une feuille de séparation optionnelle 14, un ensemble de seringues 20 présentant des flasques 21, un plateau-nest 30 et une boîte 40. Suivant l'invention, le couvercle 32 présente une paroi supérieure 34, et des flancs 24, 24' qui s'étendent vers le bas, de deux cotés opposés de la paroi supérieure 34. Le plateau représenté dans les exemples qui suivent est un plateau-nest. L'invention peut cependant également être utilisée en conjonction avec un plateau-peigne. Dans la suite, on utilisera le terme générique « plateau » pour désigner ces deux types de plateaux.

Les Figs. 1b et 1c représentent un mode de solidarisation du couvercle 32 à un plateau 30. L'assemblage désassemblage d'un couvercle 32 sur un plateau 30 peut se faire très aisément par des moyens automatiques : par exemple, un outil de préhension d'un robot comporte une pluralité de ventouses qui s'appliquent sur la face supérieure de la paroi supérieure 34. L'outil de préhension imprime une courbure en « u » à la paroi supérieure 34, comme représenté schématiquement à la Fig. 1 b. Les doigts opposés 36, 36' sont alors légèrement écartés, et peuvent s'agencer sous le plateau 30. L'outil de préhension libère alors le couvercle 32, qui reprend sa forme plane et est alors solidarisé au plateau 30. Les mêmes opérations en sens inverse permettent de désolidariser le couvercle 32 du plateau 30. En variante, le couvercle 32, non courbé, peut être glissé longitudinalement sur le plateau. Ce mode de solidarisation est avantageux lorsque le couvercle présente une forme de glissière, ou une forme extrudable.

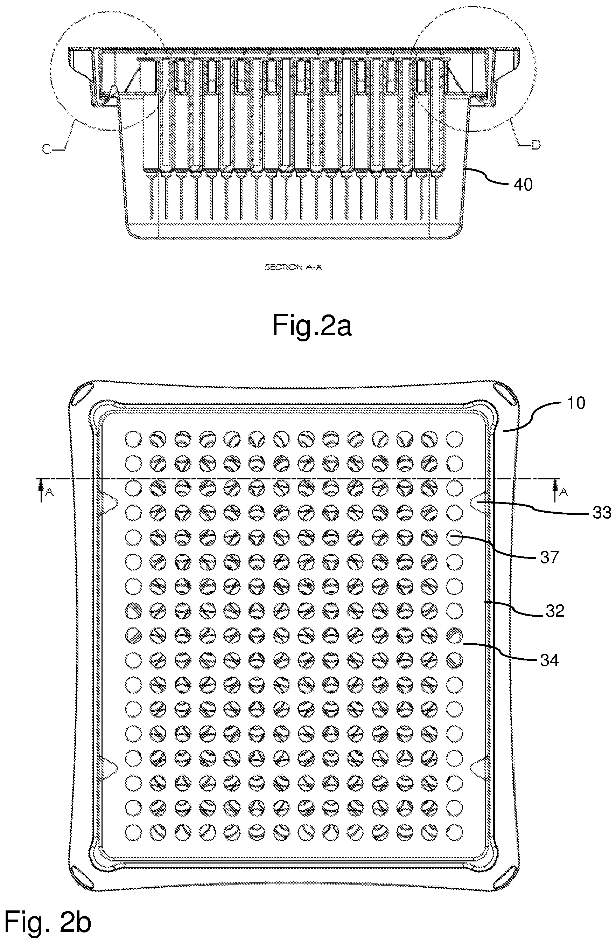

La Fig. 2a représente le couvercle d'un premier mode de réalisation de l'invention, et indique comment les doigts horizontaux 36 et 36' prolongeant les flancs 24, 24' s'insèrent sous la face inférieure du plateau 30. Ces doigts horizontaux s'étendent sensiblement sur toute la longueur des flancs 24, 24'. Le couvercle 32 peut être réalisé par moulage ou même par extrusion d'un polymère tel que du polycarbonate. Les seringues 20 sont supportées par leurs flasques 21 de telle sorte que leurs aiguilles soient suspendues au dessus du fond 43 de la boîte 40. La boîte 40 comporte de manière connue un redan 45 destiné à supporter le plateau 30 à une hauteur appropriée. Suivant un mode de réalisation préféré de l'invention, ce redan peut comporter, à sa partie située vers l'intérieur de la boîte, une nervure en forme de « U » inversé 41, sur tout ou partie du pourtour du redan 45. Cette nervure 41 a pour effet que les extrémités périphériques du plateau 30 sont libres, et donc que les doigts 36, 36' peuvent venir s'agencer sous le plateau 30 même lorsque celui-ci est posé dans la boîte : il n'est pas nécessaire de soulever le plateau 30 pour agencer le couvercle 32.

La Fig. 2b représente notamment des ouvertures 37 pratiquées dans la paroi supérieure 34 du couvercle 32. Ces ouvertures 37 permettent la circulation de la vapeur ou du gaz lors de la stérilisation. Dans un mode de réalisation préféré, elles sont agencées au dessus des ouvertures des contenants 20 et permettent ainsi le remplissage et/ou le bouchonnage de ceux-ci. Des échancrures de centrage 33 peuvent être prévues dans la paroi supérieure 34 du couvercle 32. Ces échancrures de centrage peuvent être utilisées par des moyens automatiques de préhension pour positionner avec précision le couvercle lors des opérations industrielles.

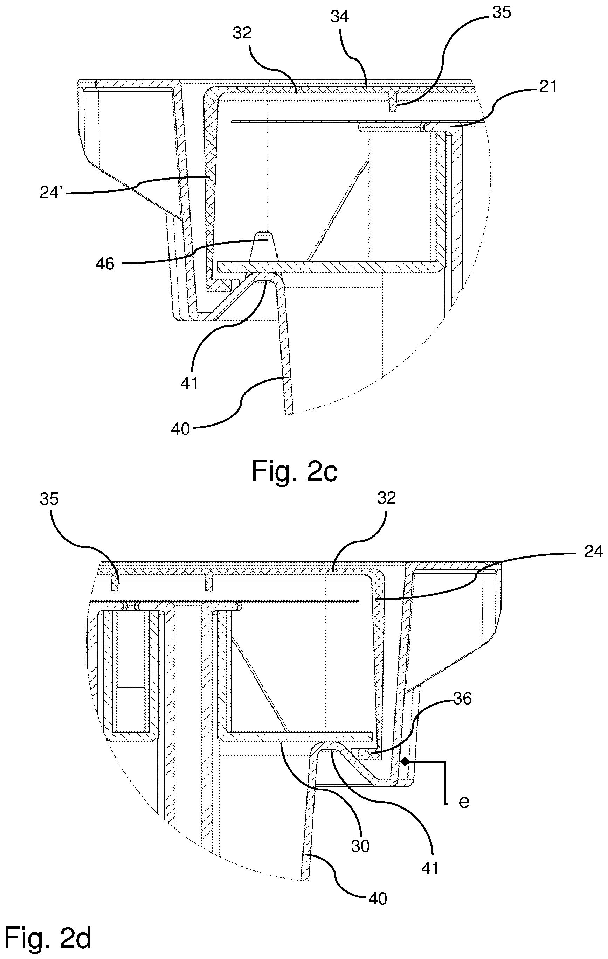

La Fig. 2d montre que le plateau 30 repose sur le sommet de la nervure en forme de « U » inversé 41. Le couvercle 32 repose sur le versant extérieur de la nervure en forme de « U » inversé 41 et laisse un espace indiqué par la lettre « e » entre le flanc 24 du couvercle et la paroi latérale de la boîte, de sorte que l'on puisse mettre/enlever le couvercle 32 lorsque le plateau 30 se trouve dans la boîte 40.

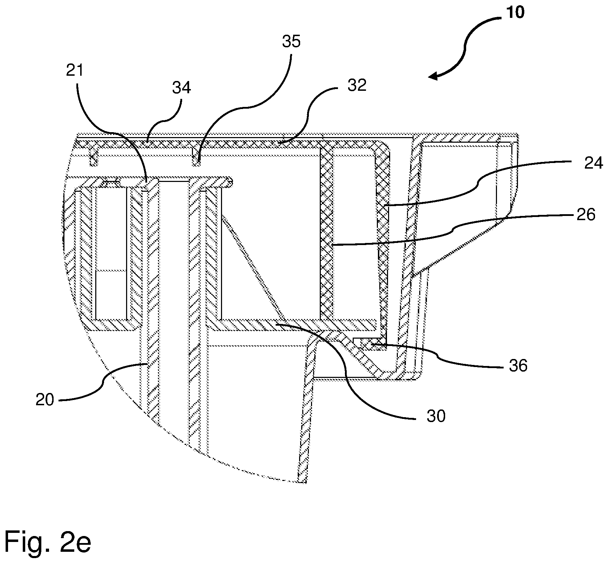

Dans un second mode de réalisation de l'invention, représenté à la Fig 2e, le couvercle 32 comporte en outre des parois de support 26, 26' parallèlement aux flancs 24, 24' et situés du coté intérieur de ceux-ci. Ces parois de support 26, 26' constituent un appui du couvercle 32 sur la périphérie du plateau 30. Elles ont une hauteur telle que ni la paroi supérieure 34 ni les nervure 35 ne s'appuient sur la face supérieure des flasques 21 des contenants 20. On peut ainsi empiler un nombre de conditionnements 10, sans risquer d'appliquer des efforts sur les contenants 20.

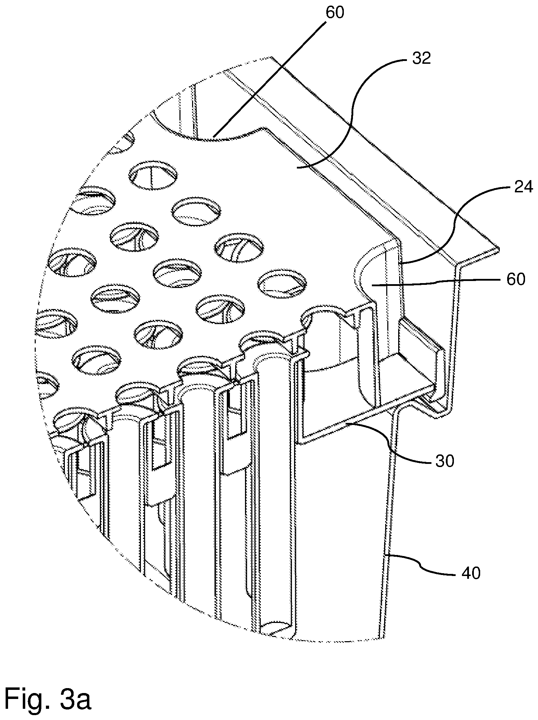

La Fig. 3a est une vue en perspective et en coupe d'un conditionnement comportant un couvercle 32 suivant un troisième mode de réalisation de l'invention. Suivant ce mode, les flancs 24, 24' sur deux cotés du couvercle 32 sont interrompus et agencés à des portions de tube 60. Ces portions de tube jouent le rôle des parois de support 26, 26' du second mode de réalisation : elles permettent l'appui du couvercle 32 sur le plateau 30. Un avantage de ce troisième mode de réalisation est que ces portions de tube permettent l'accès par le dessus à une portion de la face supérieure du plateau 30. Des moyens de préhension automatiques, munis par exemple de ventouses, peuvent alors déposer ou prendre l'ensemble couvercle/plateau dans une boîte 40.

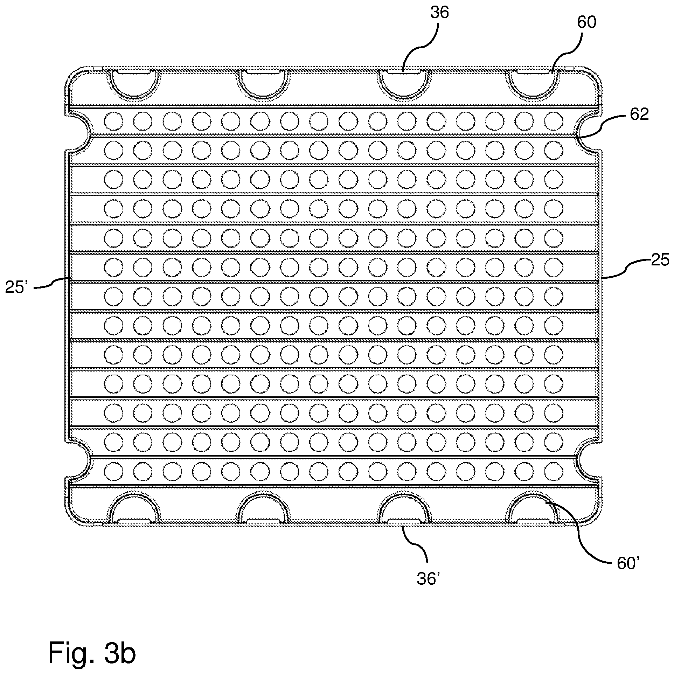

La Fig. 3b est une vue de dessous d'un couvercle 32 suivant le troisième mode de réalisation. Des portions de tube 62 sont également prévus sur les deux autres cotés du couvercle 32, ce qui a pour effet de faciliter la flexion du couvercle, malgré la présence de flancs 25, 25' sur ces cotés.
Les doigts 36, 36' sont disposés au niveau des tubes des portions de tube 60, 60', ce qui permet la réalisation d'un moule de fabrication simple.

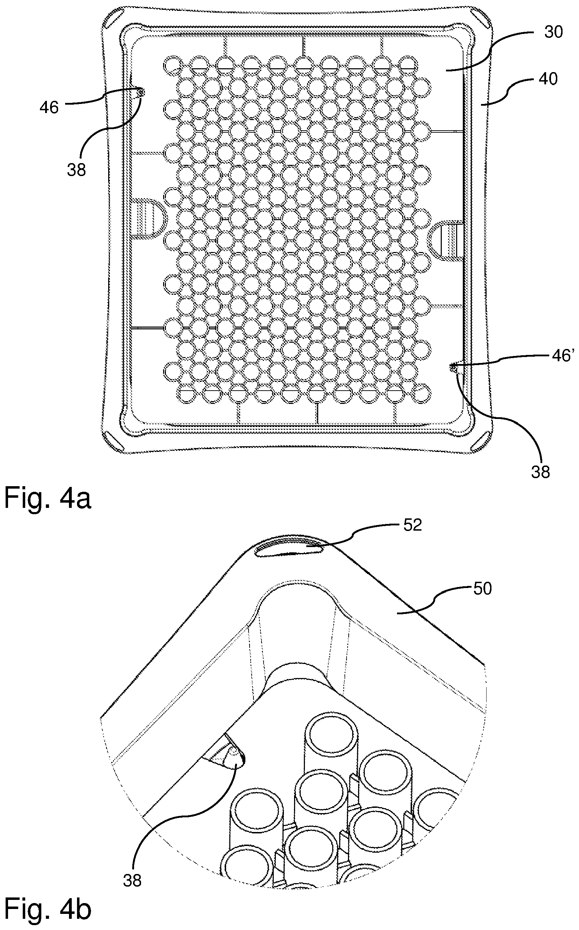

La Fig. 4a est une vue de dessus d'une boîte 40 contenant un plateau 30 d'un conditionnement suivant l'invention, et montre la position moyens d'immobilisation du plateau 30 dans la boîte 40. Suivant des premiers moyens d'immobilisation, représentés aux Fig. 4a et 4b, des plots de centrage 46, 46' sont prévues sur le redan 45 de la boîte, et des échancrures d'immobilisation 47, 47' correspondantes sont prévues dans le plateau 30.

La Fig. 2c est une vue agrandie de la coupe A-A qui montre le plot de centrage 46. On voit sur cette Fig. 2c que ce plot de centrage présente une forme tronconique. Par ailleurs, on voit sur les Fig. 4a et 4b que l'échancrure 47 présente une forme en « V ». Ces forment assurent d'une part, que lors du dépôt du plateau dans la boîte le plateau se centre automatiquement dans la boîte. D'autre part, ces formes assurent un serrage qui évite tout mouvement, générateur de particules lors du stockage et/ou du transport.

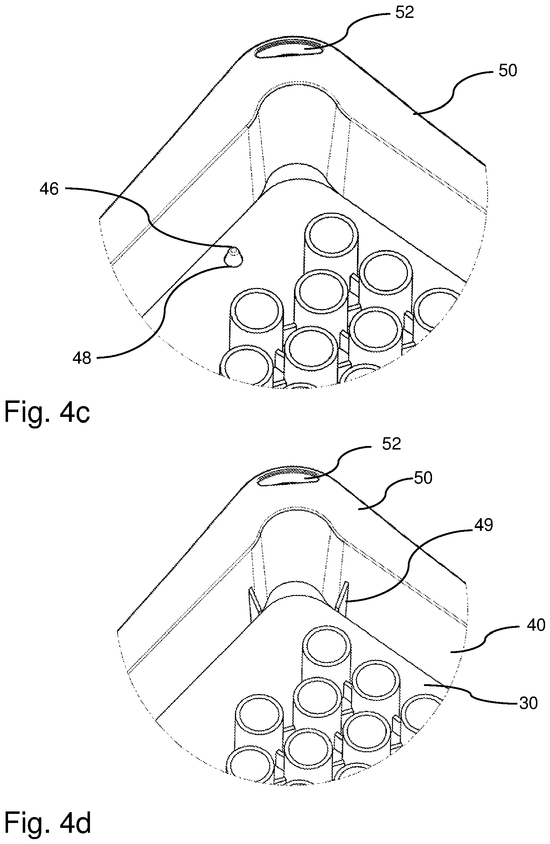

La Fig. 4c représente des seconds moyens d'immobilisation, dans lesquels l'échancrure d'immobilisation 47 est remplacée par un simple trou rond 48 prévu dans le plateau 30.

La Fig. 4d représente des troisièmes moyens d'immobilisation, dans lesquels une nervure 49 est agencée verticalement le long de la face interne de la paroi latérale supérieure de la boîte 40. Cette nervure peut avantageusement présenter un profil incliné qui guide progressivement le plateau 30 en position. Ces troisièmes moyens d'immobilisation présentent l'avantage qu'ils peuvent être utilisés avec des plateaux-nests non modifiés, et qui constituent un standard industriel.

On positionne avantageusement deux moyens d'immobilisation sur des cotés opposés et proches d'angles opposés de la boîte, comme représenté à la Fig. 4a. L'utilisation des ces moyens d'immobilisation assure qu'un plateau 30 posé dans une boîte 40 est localisé à un endroit précis, et n'a aucun degré de liberté. Il n'est donc pas susceptible de se déplacer lors du transport et/ou du stockage. On évite ainsi la création de particules. Par ailleurs, la connaissance précise de la position du plateau 30 dans la boîte 40 peut faciliter certaines opérations telles que le remplissage des contenants et la préhension automatique. On comprendra qu'il est possible deux ou plus de deux moyens d'immobilisation et de combiner les premier, second et troisièmes moyens.

Les Fig. 4a, 4b et 4c représentent une caractéristique optionnelle de la boîte 40. Dans une telle boîte, il est connu de prévoir un rebord supérieur 50, qui permet le collage d'un opercule 12, représenté à la Fig. 1a. Dans un mode préféré de l'invention, on a prévu une ouverture 52 dans au moins un coin de ce rebord 50. Cette ouverture permet l'écoulement de vapeur d'eau condensée ou de gaz, à l'issue de la stérilisation. De plus, il permet de soulever un opercule 12 afin de pouvoir le décoller aisément.

Comme montré en agrandissement aux Figs. 2c, 2d et 2e, la paroi supérieure 34 du couvercle 32 peut comprendre à sa face inférieure, des nervures 35. Ces nervures permettent d'assurer la rigidité du plateau, et sont de préférence orientées parallèlement aux flancs 24, 24', de manière à maintenir une certaines flexibilité du plateau dans le sens perpendiculaire aux flancs 24, 24'. De préférence, ces nervures 35 sont prévues au dessus des flasques 21 des contenants 20, pour optimiser les procédés de stérilisation des seringues 20 de la manière suivante. Comme le plateau 30 peut être retourné sans laisser tomber les seringues 20, il est possible de réaliser la stérilisation, telle que la stérilisation à la vapeur, en gardant les seringues 20 dans une position verticale renversée. L'avantage de procéder à une stérilisation des seringues dans une position verticale inversée est d'éviter l'accumulation d'eau de condensation dans les seringues. Les résidus de gaz de stérilisation peuvent également être évacués. Lesdites nervures 35 séparent les flasques 21 des seringues 20 inversées de la paroi supérieure 34 dudit plateau 30, ce qui a pour conséquence que la stérilisation est plus efficace car la vapeur ou le gaz peut aisément atteindre les parties internes de la seringue 20.

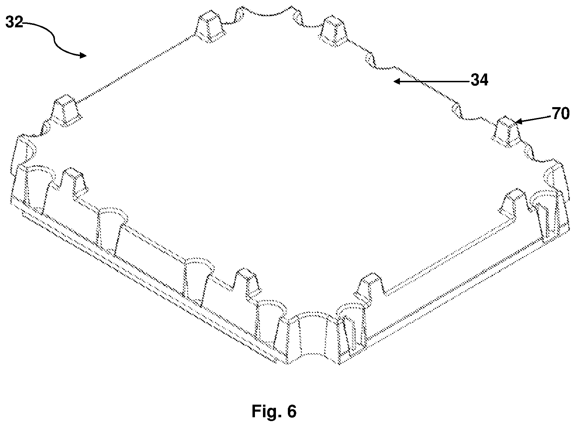

La Fig. 6 représente une vue en perspective d'un couvercle 32 selon un mode de réalisation particulier préféré de l'invention. Le couvercle 32 comprend en outre sur sa paroi supérieure 34, un moyen de positionnement 70 d'un second conditionnement 10' (non représenté). Comme représenté à la Fig. 6, le moyen de positionnement 70 peut être une saillie ou un plot. Une pluralité de plots ou de saillies peut être disposée sur la paroi supérieure 34 du couvercle 32 afin de stabiliser le second conditionnement 10' positionné sur ledit couvercle 32. Ainsi, plusieurs conditionnements 10,10' peuvent être superposés mais garder une stabilité nécessaire pour éviter tout risque lors de son transport. Alternativement, le moyen de positionnement 70 peut être une jupe continue disposée sur le pourtour de la paroi supérieure 34.

Le conditionnement suivant l'invention permet, grâce à son couvercle, d'éviter tout contact direct avec les contenants et leurs flasques. Il permet l'empilement de conditionnements, à divers stades de leur fabrication, transport, contrôle. Il permet également de stériliser les contenants après leur remplissage (la « post-stérilisation ») sans nécessiter de désassemblage/réassemblage des conditionnements. La fabrication du couvercle est peu onéreuse, particulièrement lorsqu'il est réalisé par injection d'un polymère dans un moule simple. On peut donc en prévoir un pour chaque contenant, utilisé tout au long de la chaîne de fabrication.

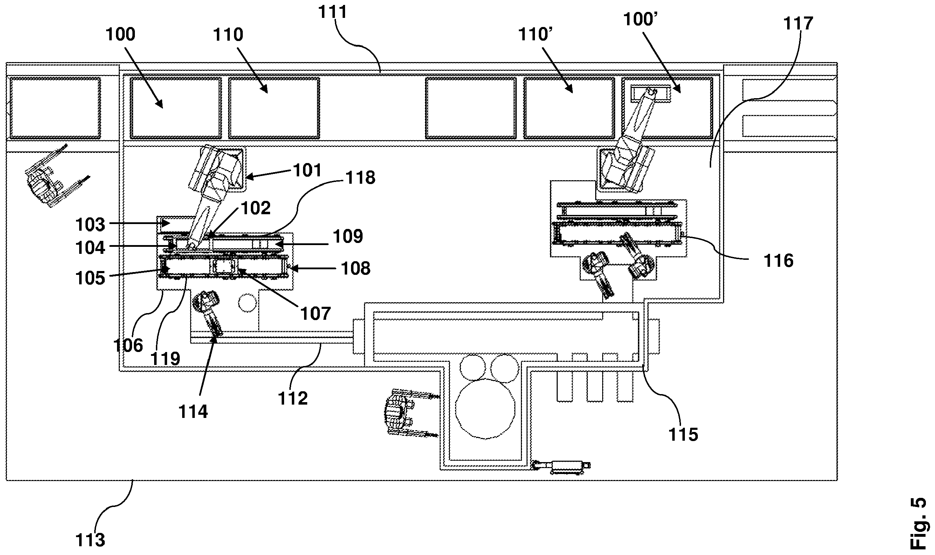

La Fig. 5 représente une vue schématique d'une zone de manipulation d'un conditionnement selon un mode de réalisation suivant l'invention. La zone de manipulation 113 peut être, par exemple, une zone d'inspection optique d'objets 20 contenus dans un plateau 30 ou une zone de remplissage des objets 20 par une solution contenant un agent thérapeutique. Cette zone de manipulation 113 comprend notamment un convoyeur de palettes 111 sur lequel transitent des palettes pleines 100, 100' et/ou vides 110, 110', un premier robot de préhension 101, un dispositif de dénestage 106 et un convoyeur 112 d'objets 20. Par « dénestage » on entend l'opération de démontage d'un conditionnement 10 afin d'obtenir des objets 20 séparés des autres éléments constituant le conditionnement 10. Le dispositif de dénestage 106 peut comprendre un magasin de couvercles 103, un convoyeur 118 de boîtes 40, un convoyeur 119 de plateau 30 et une unité de dénestage 107. Le dispositif de dénestage 106 permet de séparer les éléments constitutifs d'un conditionnement 10 quelque soit le type de plateau utilisé. L'unité de dénestage 107 permet d'enlever les objets 20 du plateau 30 que ce dernier soit un plateau de type nest ou peigne. Ainsi, à la sortie du dispositif de dénestage 106, les objets 20 sont envoyés par l'intermédiaire d'un second robot préhenseur 114 et d'un convoyeur d'objets 112 vers une seconde zone de traitement 115 telle que par exemple une zone d'inspection optique.

Selon un mode de réalisation particulier de l'invention, les conditionnements 10 selon l'invention, disposés dans la palette 100, comprennent un plateau 30 supportant des objets 20, une boîte 40 et un couvercle 32 tel que décrit précédemment (non représenté sur la Fig. 5). En particulier, le couvercle 32 possède sur deux côtés des flancs 24,24' interrompus et agencés à des portions de tube 60 comme représenté à la Fig. 3a. Ces portions de tube 60 permettent l'appui du couvercle 32 sur le plateau 30. Le couvercle comprend également des portions de tube 62 sur les deux autres côtés, facilitant ainsi la flexion du couvercle.

Une palette vide 110 est placée à l'entrée du convoyeur 111 puis déplacée en face du premier robot de préhension 101. Une palette pleine 100 comprenant une pluralité de conditionnements 10 est placée à l'entrée du convoyeur 111 puis placée en face du premier robot de préhension 101 comme représenté à la Fig. 5. Le terme « en face du premier robot de préhension 101 » signifie que lesdites palettes 110 et/ou 100 se trouve dans un périmètre permettant audit premier robot de préhension 101 d'effectuer une ou plusieurs manipulations sur celles-ci. Par exemple, le premier robot de préhension 101 peut donc prendre un conditionnement 10 dans la palette pleine 100 ou déposer un conditionnement 10 dans une palette vide 110. Selon un mode de réalisation, le premier robot de préhension 101 saisit un ou plusieurs conditionnements 10 par l'intermédiaire de son préhenseur 102. De préférence, le premier robot de préhension 101 saisit deux conditionnements 10,10' simultanément. Lesdits un ou plusieurs conditionnements 10 sont déplacés vers le dispositif de dénestage 106 où la séparation des éléments constitutifs du conditionnement 10 s'effectue. Les étapes suivantes du procédé de séparation seront décrites en considérant que le robot de préhension 101 manipule deux conditionnements 10 et 10' simultanément. Il est évident à la lecture de la description que les étapes suivantes du procédé peuvent également être effectuées lorsque le premier robot de préhension 101 manipule un seul conditionnement 10 ou plus de deux conditionnements 10, par exemple trois, quatre, cinq ou six conditionnements simultanément.

Les conditionnements 10,10' (non représentés sur le Fig. 5) saisis par le robot de préhension 101 sont déplacés à la verticale de la première extrémité 104 du convoyeur 118. Les boîtes 40,40' desdits conditionnements 10,10' sont déposées sur le convoyeur 118 au niveau de ladite extrémité 104. La séparation des boîtes 40,40' du reste des conditionnements 10,10', c'est-à-dire de l'ensemble solidaire formé par un plateau 30 et un couvercle 32, est effectuée par le préhenseur 102 dudit premier robot de préhension 101. Le préhenseur 102 peut imposer une pression sur la surface latérale des boîtes 40,40' et ainsi favoriser la séparation desdites boîtes 40,40' des ensembles couvercle/plateau 32,30 et 32',30'. Grâce à la présence des portions de tube 60 sur chacun des couvercles 32,32' de chacun des conditionnements 10,10', le premier robot de préhension 101 peut avoir accès à une portion de la face supérieur des plateaux 30,30' et ainsi maintenir les ensembles couvercle/plateau 32,30 et 32',30' solidaires. Le préhenseur 102 munis par exemple de ventouses peut saisir, pour chacun des conditionnements 10,10', l'ensemble couvercle/plateau 32,30 et 32',30' et les désolidariser des boîtes 40,40'. Cette étape est effectuée avec un seul robot de préhension 101 contrairement au procédé de l'art antérieur. En effet, les conditionnements utilisés actuellement comprennent un couvercle muni de doigts se fixant sous le rebord de la boîte de conditionnement. Cette configuration nécessite l'emploi d'un second robot ou une manipulation manuelle pour tenir la boîte lorsque le couvercle est enlevé. Lesdites boîtes 40,40' peuvent être disposés sur un premier support mobile apte à déplacer celles-ci sur le convoyeur 118 de la première extrémité 104 et la seconde extrémité 109.

L'ensemble couvercle/plateau 32,30 de chacun des conditionnements 10,10' est placé à la verticale de la première extrémité 105 du convoyeur 119. Les plateaux 30,30' sont déposés sur le convoyeur 119 au niveau de la première extrémité 105. Les plateaux 30,30' peuvent être déposés sur un second support mobile apte à déplacer ceux-ci le long du convoyeur 119 de la première extrémité 105 et la seconde extrémité 108. L'action du préhenseur 102 dudit premier robot de préhension 101 permet de séparer les couvercles 32,32' des plateaux 30,30' par l'imposition d'une courbure en « u » à chacune des parois supérieures des couvercles 32,32'. Les plateaux 30,30' sont ainsi désolidarisés des couvercles 32,32'. Les couvercles 32,32' peuvent être déplacés à la verticale du magasin de couvercle 103 et déposés à l'intérieur de celui-ci par le préhenseur 102 du premier robot de préhension 101. Lors du traitement d'une palette 100 comprenant plus de quatre conditionnements, seuls les quatre premiers couvercles 32 issus des quatre premiers conditionnements 10 sont disposés à l'intérieur du magasin de couvercle 103. Dans les autres cas, le préhenseur 102 maintenant les couvercles 32,32' se positionne à la verticale de la seconde extrémité 108 du convoyeur de plateau 119.

Les étapes visant au retrait des objets 20,20' contenus respectivement dans les plateaux 30,30' sont ensuite engagées. Les boîtes 40,40' disposées à la première extrémité 104 du convoyeur de boîtes 118 sont déplacées vers la seconde extrémité 109 de ce même convoyeur 118. Les plateaux 30,30' positionnés au niveau de la première extrémité 105 du convoyeur de plateau 119 sont déplacés au niveau l'unité de dénestage 107. Les objets 20,20' sont retirés des plateaux 30,30' au niveau de cette unité de dénestage 107. Les objets 20,20' peuvent être pris en charge par un second robot de préhension 114 et disposés sur un convoyeur d'objets 112. Les plateaux 30,30' vides sont ensuite déplacés de l'unité de dénestage 107 vers la seconde extrémité 108 du convoyeur de plateau 119. Les plateaux 30,30' se situent ainsi à l'aplomb du préhenseur 102 contenant les couvercles 32,32'. Néanmoins, pour les quatre derniers conditionnements de la palette 100 traitée dans la zone de manipulation 113, le premier robot de préhension 101 se positionne à la verticale du magasin de couvercle 103 pour se saisir de deux couvercles 32,32' puis se positionne à la verticale de la seconde extrémité 108 du convoyeur 119. Les plateaux 30,30' sont assemblés avec les couvercles 32,32'. L'assemblage s'effectue par l'intermédiaire du robot de préhension 101 et de son préhenseur 102. Ledit préhenseur 102 impose une courbure ou une déformation aux parois supérieures des couvercles 32,32', permettant ainsi aux doigts 36,36', situés aux extrémités inférieures des flancs 24,24', de chacun des couvercles 32,32' de s'agencer sous une face inférieure desdits plateaux 30,30' solidarisant respectivement les plateaux 30,30' et les couvercles 32,32'. Ledit préhenseur 102 maintient donc les ensembles plateaux/couvercles 30/32 et 30'/32' par l'intermédiaire de leurs portions de tube respectives 60. Le préhenseur 102 se positionne à la verticale de la seconde extrémité 109 du convoyeur 118 et assemble les ensembles plateaux/couvercles 30/32 et 30'/32' respectivement avec les boîtes 40 et 40'. Les conditionnements 10,10' vidés des objets 20,20' sont ainsi reconstitués. Les conditionnements 10,10' vidés des objets 20,20' sont déplacés à la verticale du convoyeur à palette 111 puis déposés dans une palette vide 110.

Alternativement, selon un autre mode de réalisation de l'invention, le convoyeur de boîtes 118 peut être remplacé par un magasin de boîtes. Les boîtes 40 sont ainsi déposées sur une surface non mobile du dispositif de dénestage 106. Lorsque les opérations de vidange ou d'assemblage du plateau ont été effectuées, le robot de préhension positionne l'ensemble plateau/couvercle 30/32 dans la boîte correspondante, saisit cette dernière et la déplace vers le convoyeur à palette 111 pour la positionner à l'intérieur d'une palette 110.

Les opérations peuvent être répétées jusqu'à ce que la palette pleine 100 soit vidée de tous ses conditionnements 10. Comme représenté sur le Fig. 5, la palette 110 peut être déplacée via la convoyeur de palette 111 dans une zone de préparation 117 et disposé en position 110'. Cette palette 110' comprend donc des conditionnements 10 dont le plateau 30 a été vidé des objets 20. La méthode de séparation tel que décrite précédemment peut également être effectuée dans la zone de préparation 117. Seule l'unité de dénestage 107 du dispositif de dénestage 106 peut être remplacée par un dispositif d'assemblage 116 des objets 20 dans le plateau 30 effectuant ainsi les opérations inverses de l'opération de dénestage. La palette 100' est ainsi remplie avec des conditionnements 10 comprenant des objets 20 maintenus en position verticale dans le plateau 30.

Les termes et descriptions utilisés ici sont proposés à titre d'illustration seulement et ne constituent pas des limitations. L'homme du métier reconnaîtra que de nombreuses variations sont possibles dans l'esprit et la portée de l'invention telle que décrite dans les revendications qui suivent et leurs équivalents; dans celles-ci, tous les termes doivent être compris dans leur acception la plus large à moins que cela ne soit indiqué autrement.

## Revendications

1. Conditionnement (10) pour le stockage et/ou le transport et/ou le traitement et/ou le support dans une position verticale suspendue d'objets (20) qui présentent des flasques (21), ledit conditionnement comprenant une boîte (40) avec une ouverture supérieure (42), un fond (43), des parois latérales (44), un plateau (30), apte à maintenir une pluralités des dits objets dans une position verticale, un redan (45) formé entre ladite ouverture supérieure (42) et lesdites parois latérales (44) pour supporter ledit plateau (30), et un couvercle (32) apte à recouvrir ledit plateau,
**caractérisé en ce que** ledit couvercle (32) comprend une paroi supérieure (34) et au moins deux flancs (24, 24') s'étendant vers le bas de deux cotés opposés de la paroi supérieure (34), chacun des flancs (24, 24') étant prolongés, à leurs extrémités inférieures, de doigts (36, 36') aptes à s'agencer sous une face inférieure dudit plateau (30), solidarisant ainsi le plateau (30) et le couvercle (32), et **en ce que** ledit redan comporte une nervure en forme de « U » inversé (41), s'étendant sur la portion intérieure dudit redan (45).

2. Conditionnement suivant la revendication 1 **caractérisé en ce que** ladite paroi supérieure (34) présente une flexibilité permettant de l'incurver de manière à écarter un doigt (36) d'un doigt (36') opposé, et permettre ainsi l'agencement de ces doigts (36, 36') sous la face inférieure dudit plateau (30).

3. Conditionnement suivant la revendication 1 ou 2 **caractérisé en ce que** le couvercle (32) comprend sur sa paroi supérieure (34) des moyens de positionnement (70) d'un second conditionnement (10').

4. Conditionnement suivant l'une quelconque des revendications précédentes **caractérisé en ce que** ladite paroi supérieure (34) présente une pluralité de passages (37).

5. Conditionnement suivant l'une quelconque des revendications précédentes **caractérisé en ce que** ladite paroi supérieure (34) comporte sur sa face inférieure, des nervures (35).

6. Conditionnement suivant l'une quelconque des revendications précédentes **caractérisé en ce que** ladite paroi supérieure (34) comporte sur sa face inférieure, au moins deux parois de support (26, 26') s'étendant vers le bas, parallèlement aux flancs (24, 24') et situés du coté intérieur de ceux-ci.

7. Conditionnement suivant l'une quelconque des revendications précédentes **caractérisé en ce que** les flancs (24, 24') sont interrompus, et que des portions de tube (60, 60') sont agencés dans ces interruption.

8. Conditionnement (10) suivant la revendication 1 **caractérisé en ce que** ledit redan (45) comporte au moins un plot de centrage (46, 46') et que le plateau (30) comporte au moins une échancrure (47, 47') ou un trou rond (48) correspondant.

9. Conditionnement (10) suivant l'une quelconque des revendications 1 à 8 **caractérisé en ce que** le ou les plots (46,46') ont une forme tronconique et/ou le ou les échancrures (47, 47') présentent une forme en « V ».

10. Conditionnement (10) suivant l'une quelconque des revendications 1 à 9 **caractérisé en ce que** la face interne de la paroi latérale supérieure de la boîte comporte une nervure verticale (49).

11. Conditionnement (10) suivant l'une quelconque des revendications 1 à 10 dans lequel ladite ouverture supérieure (42) de ladite boîte (40) comporte un rebord supérieur (50) caractérisé en ce ledit rebord supérieur (50) comporte, dans au moins un angle de ladite boîte (40) une ouverture (52).

12. Méthode de séparation et/ou d'assemblage des éléments constitutifs d'un ou plusieurs conditionnements (10) comprenant un plateau (30), apte à maintenir une pluralités d'objets (20) dans une position verticale, lesdits objets (20) présentant des flasques (21), un couvercle (32) apte à recouvrir ledit plateau (30), et une boîte (40) supportant ledit plateau, ladite méthode comprenant les étapes de :
- préhension d'un ou plusieurs conditionnements (10) selon l'une quelconque des revendications 1 à 11 par un moyen de préhension automatique,
- séparation de ladite boîte (40) de l'ensemble formé par le plateau (30) recouvert du couvercle (32) par l'intermédiaire dudit moyen de préhension automatique,
- séparation dudit plateau (30) contenant lesdits objets (20), dudit couvercle (32) par le moyen de préhension automatique,
- traitement dudit plateau (30), de préférence l'étape de traitement dudit plateau (30) comprend le retrait des objets (20) maintenus en position verticale dans ledit plateau (30) pour mettre ceux-ci à disposition ou la disposition d'objets (20) munis de flasques (21) dans ledit plateau (30),
- réassemblage successif dudit plateau (30) avec ledit couvercle (32) puis de l'ensemble ainsi formé, avec ladite boîte (40) par le moyen de préhension automatique.

13. Méthode selon la revendication 12 **caractérisée en ce qu'**elle comporte l'utilisation d'un couvercle (32) comprenant une paroi supérieure (34) et au moins deux flancs (24,24') s'étendant vers le bas de deux cotés opposés de la paroi supérieure (34), chacun des flancs (24,24') étant prolongés, à leurs extrémités inférieures, de doigts (36,36') aptes à s'agencer sous une face inférieure dudit plateau (30), solidarisant ainsi le plateau (30) et le couvercle (32), et **en ce que** lesdits flancs (24,24') sont interrompus, et que des portions de tube (60,60') sont agencés dans ces interruptions.

14. Procédé de stockage et/ou de transport et/ou de traitement et/ou de support d'objets (20) suspendus en position verticale et présentant des flasques (21) comprenant l'utilisation d'un plateau (30), apte à maintenir une pluralités desdits objets dans une position verticale, et un couvercle (32) apte à recouvrir ledit plateau, comportant les étapes de
- mise à disposition d'un couvercle (32) comprenant une paroi supérieure (34) et au moins deux flancs (24, 24') s'étendant vers le bas de deux cotés opposés de la paroi supérieure (34), chacun des flancs (24, 24') étant prolongés, à leurs extrémités inférieures, de doigts (36, 36') aptes à s'agencer sous une face inférieure dudit plateau (30),
- préhension dudit couvercle (32) par des moyens de préhension ;
- imposition par les moyens de préhension d'une courbure à la paroi supérieure du couvercle (34) écartant deux flancs (24, 24') l'un de l'autre ;
- positionnement du couvercle (32) en regard du plateau (30), le plateau étant supporté dans une boîte comportant une ouverture supérieure (42), un fond (43), des parois latérales (44), un redan (45) formé entre ladite ouverture supérieure (42) et lesdites parois latérales (44) pour supporter ledit plateau (30) et comportant une nervure en forme de « U » inversé (41), s'étendant sur sa portion intérieure;
- relâchement de la dite courbure, les doigts (36, 36') solidarisant ainsi le plateau (30) et le couvercle (32).

## Patentansprüche

1. Aufbewahrungssystem (10) für die Lagerung und/oder den Transport und/oder die Behandlung und/oder die Halterung in einer senkrechten, aufgehängten Position von Objekten (20), welche Flansche (21) aufweisen, wobei das Aufbewahrungssystem einen Kasten (40) mit einer oberen Öffnung (42), einen Boden (43), Seitenwände (44), ein Tablett (30), welches geeignet ist, eine Vielzahl der Objekte in einer senkrechten Position zu halten, eine zwischen der oberen Öffnung (42) und den Seitenwänden (44) ausgebildete Unterschneidung (45), um das Tablett (30) zu tragen, und einen Deckel (32), welcher geeignet ist, das Tablett abzudecken, aufweist,
**dadurch gekennzeichnet, dass** der Deckel (32) eine obere Wand (34) und mindestens zwei Flansche (24, 24`) aufweist, welche sich an zwei gegenüber liegenden Seiten der oberen Wand (34) nach unten erstrecken, wobei jeder der Flansche (24, 24`) an seinem unteren Ende von Fingern (36, 36`) verlängert wird, welche geeignet sind, sich unter einer unteren Fläche des Tabletts (30) anzuordnen und so das Tablett (30) und den Deckel (32) fest zu verbinden, und dass die Unterschneidung eine Rippe in Form eines umgekehrten "U" (41) aufweist, welche sich über den inneren Abschnitt der Unterschneidung (45) erstreckt.

2. Aufbewahrungssystem nach Anspruch 1, **dadurch gekennzeichnet, dass** die obere Wand (34) eine Flexibilität aufweist, welche es ermöglicht, sie zu biegen, um einen Finger (36) von einem gegenüber liegenden Finger (36`) weg zu drücken und so die Anordnung dieser Finger (36, 36`) unter der unteren Fläche des Tabletts (30) anzuordnen.

3. Aufbewahrungssystem nach Anspruch 1 oder 2, **dadurch gekennzeichnet, dass** der Deckel (32) auf seiner oberen Wand (34) Mittel zur Positionierung (70) eines zweiten Aufbewahrungssystems (10`) aufweist.

4. Aufbewahrungssystem nach einem der vorhergehenden Ansprüche, **dadurch gekennzeichnet, dass** die obere Wand (34) eine Vielzahl von Durchlässen (37) aufweist.

5. Aufbewahrungssystem nach einem der vorhergehenden Ansprüche, **dadurch gekennzeichnet, dass** die obere Wand (34) auf ihrer unteren Fläche Rippen (35) aufweist.

6. Aufbewahrungssystem nach einem der vorhergehenden Ansprüche, **dadurch gekennzeichnet, dass** die obere Wand (34) auf ihrer unteren Fläche mindestens zwei Stützwände (26, 26') aufweist, welche sich parallel zu den Flanschen (24, 24') nach unten erstrecken und sich auf deren inneren Seite befinden.

7. Aufbewahrungssystem nach einem der vorhergehenden Ansprüche, **dadurch gekennzeichnet, dass** die Flansche (24, 24`) unterbrochen sind und dass Rohrabschnitte (60, 60`) in diesen Unterbrechungen angeordnet sind.

8. Aufbewahrungssystem (10) nach Anspruch 1, **dadurch gekennzeichnet, dass** die Unterschneidung (45) mindestens einen Zentrierungszapfen (46, 46`) aufweist und dass das Tablett (30) mindestens eine entsprechende Aussparung (47, 47`) oder rundes Loch (48) aufweist.

9. Aufbewahrungssystem (10) nach einem der Ansprüche 1 bis 8, **dadurch gekennzeichnet, dass** der oder die Zapfen (46, 46`) eine kegelstumpfartige Form besitzen und/oder die Aussparung oder Aussparungen (47, 47') eine "V"-Form aufweisen.

10. Aufbewahrungssystem (10) nach einem der Ansprüche 1 bis 9, **dadurch gekennzeichnet, dass** die innere Fläche der oberen Seitenwand des Kastens eine senkrechte Rippe (49) aufweist.

11. Aufbewahrungssystem (10) nach einem der Ansprüche 1 bis 10, bei welchem die obere Öffnung (42) des Kastens (40) einen oberen Rand (50) aufweist, **dadurch gekennzeichnet, dass** der obere Rand (50) in mindestens einer Ecke des Kastens (40) eine Öffnung (52) aufweist.

12. Verfahren zum Trennen und/oder Zusammensetzen von Bestandteilen eines oder mehrerer Aufbewahrungssysteme (10), aufweisend ein Tablett (30), welches geeignet ist, eine Vielzahl von Objekten (20) in einer senkrechten Position zu halten, wobei die Objekte (20) Flansche (21) aufweisen, einen Deckel (32), welcher geeignet ist, das Tablett (30) abzudecken, und eine Kasten (40), welcher das Tablett trägt, wobei das Verfahren die folgenden Schritte aufweist:
- Ergreifen eines oder mehrerer Aufbewahrungssysteme (10) nach einem der Ansprüche 1 bis 11 durch ein automatisches Greifmittel,
- Trennen des Kastens (40) von der Baugruppe, welche von dem von dem Deckel (32) abgedeckten Tablett (30) gebildet wird, mit Hilfe des automatischen Greifmittels,
- Trennen des Tabletts (30), welches die Objekte (20) enthält, von dem Deckel (32) durch das automatische Greifmittel,
- Behandeln des Tabletts (30), vorzugsweise weist der Schritt zur Behandlung des Tabletts (30) das Entnehmen der in dem Tablett (30) in vertikaler Postion gehaltenen Objekte, um diese zur Verfügung zu stellen, oder die Anordnung von mit Flanschen (21) versehenen Objekte (20) auf dem Tablett (30) auf,
- aufeinander folgendes Wiederzusammensetzen des Tabletts (30) mit dem Deckel (32), dann der so gebildeten Baugruppe mit dem Kasten (40) durch das automatische Greifmittel.

13. Verfahren nach Anspruch 12, **dadurch gekennzeichnet, dass** es die Verwendung eines Deckels (32) umfasst, welcher eine obere Wand (34) und mindestens zwei Flansche (24, 24') aufweist, welche sich an zwei gegenüber liegenden Seiten der oberen Wand (34) nach unten erstrecken, wobei jeder der Flansche (24, 24') an seinem unteren Ende von Fingern (36, 36') verlängert wird, welche geeignet sind, sich unter einer unteren Fläche des Tabletts (30) anzuordnen und so das Tablett (30) und den Deckel (32) fest zu verbinden, und dass die Flansche (24, 24') unterbrochen sind und dass Rohrabschnitte (60, 60`) in diesen Unterbrechungen angeordnet sind.

14. Verfahren zum Lagern und/oder zum Transportieren und/oder zum Behandeln und/oder zum Halten von in einer senkrechten Position aufgehängten Objekten (20), welche Flansche (21) aufweisen, welches die Verwendung eines Tabletts (30), welches geeignet ist, eine Vielzahl von Objekten in einer senkrechten Position zu halten, und eines Deckels (32) aufweist, welcher geeignet ist, das Tablett abzudecken, aufweisend die Schritte
- Anordnen eines Deckels (32), welcher eine obere Wand (34) und mindestens zwei Flansche (24, 24`) aufweist, welche sich an zwei sich gegenüber liegenden Seiten der oberen Wand (34) nach unten erstrecken, wobei jeder der Flansche (24, 24`) an seinem unteren Ende von Fingern (36, 36`) verlängert wird, welche geeignet sind, sich unter einer unteren Fläche des Tabletts (30) anzuordnen,
- Ergreifen des Deckels (32) durch Greifmittel;
- Auferlegen der oberen Wand des Deckels (34) mit einer Biegung durch die Greifmittel, was zwei Flansche (24, 24`) von einander weg drückt;
- Positionieren des Deckels (32) gegenüber dem Tablett (30), wobei das Tablett in einem Kasten gehalten wird, welcher eine obere Öffnung (42), einen Boden (43), Seitenwände (44), eine zwischen der oberen Öffnung (42) und den Seitenwänden (44) ausgebildete Unterschneidung (45) aufweist, um das Tablett (30) zu tragen, und welche eine Rippe in Form eines umgekehrten "U" (41) aufweist, welche sich über ihren inneren Abschnitt erstreckt;
- Aufheben der Biegung, wobei so die Finger (36, 36`) das Tablett (30) und den Deckel (32) fest verbinden.

## Claims

1. Packaging (10) for the storage and/or transportation and/or processing and/or supporting in a suspended vertical position of objects (20) which have flanges (21), the said packaging comprising a tub (40) with a top opening (42), a bottom (43), lateral walls (44), a plate (30) able to hold a plurality of the said objects in a vertical position, a step (45) formed between the said top opening (42) and the said lateral walls (44) to support the said plate (30), and a lid (32) able to cover the said plate,
**characterized in that** the said lid (32) comprises a top wall (34) and at least two side walls (24, 24') extending downwards from two opposite sides of the top wall (34), each of the side walls (24, 24') being extended, at their lower ends, with fingers (36, 36') able to position themselves under an underside of the said plate (30), thus securing the plate (30) and the lid (32), and **in that** the said step comprises a rib (41) in the shape of an inverted "U", extending over the interior portion of the said step (45).

2. Packaging according to Claim 1, **characterized in that** the said top wall (34) has a flexibility that allows it to be bent so as to move a finger (36) away from an opposite finger (36') and thus allow these fingers (36, 36') to be positioned under the underside of the said plate (30).

3. Packaging according to Claim 1 or 2, **characterized in that** the lid (32) comprises on its top wall (34) positioning means (70) for positioning a second packaging (10').

4. Packaging according to any one of the preceding claims, **characterized in that** the said top wall (34) has a plurality of passages (37).

5. Packaging according to any one of the preceding claims, **characterized in that** the said top wall (34) has ribs (35) on its underside.

6. Packaging according to any one of the preceding claims, **characterized in that** the said top wall (34) comprises on its underside at least two support walls (26, 26'), extending downwards parallel to the side walls (24, 24') and situated on the inside of the latter.

7. Packaging according to any one of the preceding claims, **characterized in that** the side walls (24, 24') are interrupted and **in that** tube portions (60, 60') are arranged in these interruptions.

8. Packaging (10) according to Claim 1, **characterized in that** the said step (45) comprises at least one centring stud (46, 46') and **in that** the plate (30) comprises at least one corresponding notch (47, 47') or round hole (48).

9. Packaging (10) according to any one of Claims 1 to 8, **characterized in that** the stud or studs (46, 46') has/have a frustoconical shape and/or the notch or notches (47, 47') has/have a V-shape.

10. Packaging (10) according to any one of Claims 1 to 9, **characterized in that** the internal face of the top lateral wall of the tub has a vertical rib (49).

11. Packaging (10) according to any one of Claims 1 to 10, in which the said top opening (42) of the said tub (40) comprises a top rim (50), **characterized in that** the said top rim (50) comprises, in at least one corner of the said tub (40), an opening (52).

12. Method of separating and/or assembling the component parts of one or more packagings (10) comprising a plate (30) able to keep a plurality of objects (20) in a vertical position, the said objects (20) having flanges (21), a lid (32) able to cover the said plate (30), and a tub (40) supporting the said plate, the said method comprising the steps of:
- taking hold of one or more packagings (10) according to any one of Claims 1 to 11 using an automatic gripping means,
- separating the said tub (40) from the assembly formed by the plate (30) covered by the lid (32), using the said automatic gripping means,
- separating the said plate (30) containing the said objects (20) from the said lid (32) using the automatic gripping means,
- processing the said plate (30), the step of processing the said plate (30) preferably involving the removal of the objects (20) held in a vertical position in the said plate (30) in order to make these available or to make objects (20) equipped with flanges (21) in the said plate (30) available,
- successively reassembling the said plate (30) with the said lid (32) and then the assembly thus formed with the said tub (40), using the automatic gripping means.

13. Method according to Claim 12, **characterized in that** it involves the use of a lid (32) comprising a top wall (34) and at least two side walls (24, 24') extending downwards on two opposite sides of the top wall (34), each of the side walls (24, 24') being extended, at their lower ends, by fingers (36, 36') able to position themselves under an underside of the said plate (30), thus securing the plate (30) and the lid (32), and **in that** the said side walls (24, 24') are interrupted, and **in that** tube portions (60, 60') are arranged in these interruptions.

14. Method of storing and/or transporting and/or processing and/or supporting objects (20) suspended in a vertical position and having flanges (21), comprising the use of a plate (30) able to hold a plurality of the said objects in a vertical position, and a lid (32) able to cover the said plate, comprising the steps of
- making available a lid (32) comprising a top wall (34) and at least two side walls (24, 24') extending downwards from two opposite sides of the top wall (34), each of the side walls (24, 24') being extended, at their lower ends, by fingers (36, 36') able to position themselves under an underside of the said plate (30),
- taking hold of the said lid (32) using gripping means;
- imposing a curvature on the top wall of the lid (34), moving two side walls (24, 24') away from one another, using the gripping means;
- positioning the lid (32) to face the plate (30), the plate being supported in a tub comprising a top opening (42), a bottom (43), lateral walls (44), a step (45) formed between the said top opening (42) and the said lateral walls (44) to support the said plate (30) and comprising a rib (41) in the shape of an inverted "U", extending over its interior portion;
- releasing the said curvature, the fingers (36, 36') thus securing the plate (30) and the lid (32).
